# EUROPÄISCHE PATENTANMELDUNG

(11) **EP 0 571 880 A1**
(43) Veröffentlichungstag der Anmeldung: **01.12.1993**
(21) Anmeldenummer: 93108104.6
(22) Anmeldetag: 18.05.1993
(51) Int. Cl.: C12N 9/00, C12Q 1/68, C12N 15/52

(54) **Thermostabile Ligase aus Archaebakterien**

(30) Priorität: 23.05.1992 DE 4217134
(71) Anmelder: BOEHRINGER MANNHEIM GMBH, D-68298 Mannheim (DE)
(72) Erfinder: Kletzin, Arnulf, Dr., W-8000 München 21 (DE); Rüger, Rüdiger, Dr., W-8124 Seeshaupt (DE); Kessler, Christoph, Dr., W-8021 Dorfen (DE); Kaletta, Cortina, Dr., W-8000 München 70 (DE); Jarsch, Michael, Dr., W-8173 Bad Heilbrunn (DE)

(57) **Zusammenfassung**

Thermostabile DNA-Ligase erhältlich aus Archaebakterien und Verfahren zu ihrer Verwendung in der Nukleinsäurediagnostik

## Beschreibung

Gegenstand der Erfindung ist eine thermostabile Ligase aus Archaebakterien, Nukleinsäuren, welche für diese Ligase kodieren, und ein Verfahren zum Nachweis von Nukleinsäuren unter Verwendung dieser Ligase.

Ganz allgemein sind Ligasen Enzyme, welche bereits bestehende Enden von Polynukleotiden verknüpfen (zusammenfassend: P. Higgins, N.R. Cozzarelli 1989 in Recombinant DNA Methology S. 3-24). Bei einer Ligasereaktion wird die Bildung einer Phosphodiesterbindung zwischen einer freien 5'-Phosphatgruppe und einer im gleichen Strang eines doppelsträngigen DNA-Moleküls gegenüberliegenden, freien 3'-Hydroxylgruppe katalysiert. Die Ausbildung der Phosphodiesterbindung ist gekoppelt an die Spaltung einer Pyrophosphatbindung in einem für die Reaktion notwendigen Co-Faktor. Dabei sind sowohl intra- als auch intermolekulare Reaktionen möglich. Ligasen spielen eine wichtige Rolle bei der DNA-Replikation und bei der Reparatur von DNA-Schäden. Man unterscheidet prinzipiell zwischen zwei Typen von DNA Ligasen. Der erste Typ ist aus Eukaryonten und Bacteriophagen der T-Serie bekannt und benötigt ATP als Cofaktor. Im Gegensatz hierzu sind die Ligasen aus Eubakterien (zweiter Typ) NAD-abhängig. Bekannte Beispiele für bakterielle Ligasen sind zum einen die E-coli-Ligase, und für Phagenligasen die Ligase des Bakteriophagen T4.

Die Ligasereaktion läuft prinzipiell in drei Schritten ab:
- Adenylierung des freien Enzyms (dabei wird bei Co-Faktor NAD ein Nikotinamidmononukleotid und bei Co-Faktor ATP ein Pyrophosphat freigesetzt). Für die genannten Beispiele liegt nach dieser Reaktion AMP über die Epsilon-Gruppe eines Lysin-Restes gebunden vor.
- Der AMP-Rest wird im zweiten Schritt vom Enzym zur 5'-Phosphatgruppe des freien DNA-Endes transferiert, wobei eine energiereiche Pyrophosphatbindung gebildet wird.
- Im dritten Schritt der Ligasereaktion wird über einen nukleophilen Angriff der gegenüberliegenden 3'-Hydroxylgruppe eine Phosphatdiesterbindung ausgebildet und die AMP-Gruppe entfernt.

Eine thermostabile Ligase ist aus Thermus thermophilus (J. Biochem. 100, 123-132 (1986)) bekannt. Das Enzym hat ein Molekulargewicht von ca. 79.000 (J. Biol. Chem. Vol. 259, 16, S. 10041 bis 10047 (1984)). Dieses Enzym benötigt Magnesium ²⁺ oder Mangan ²⁺ sowie NAD als Cofaktoren, jedoch kein ATP. Das Enzym aus Thermus thermophilus HB 8 wurde auch erfolgreich in der Oligonukleotidligation der Ligase Chain Reaction (LCR) eingesetzt (WO 91/17239). Eine thermostabile, ATP-abhängige DNA-Ligase war bisher nicht bekannt.

Aufgabe der vorliegenden Erfindung war es, eine thermostabile, ATP-abhängige Ligase zu finden. Bevorzugt sollte diese die Nachteile des Standes der Technik nicht aufweisen. ATP-Abhängigkeit ist nämlich gegenüber NAD-Abhängigkeit wegen der Thermostabilität von ATP bevorzugt.

Gegenstand der Erfindung ist daher eine thermostabile Ligase erhältlich aus Archaebakterien. Ebenfalls Gegenstand der Erfindung sind Nukleinsäuresequenzen, welche für diese Ligase kodieren, ein Verfahren zum Nachweis von Nukleinsäuren mit Hilfe dieser Ligase und ein Verfahren zur Isolierung eines Enzyms.

Eine Ligase im Sinne der Erfindung ist ein Enzym, welches zwei benachbart an einen gemeinsamen Strang anhybridisierte DNA-Einzelstränge miteinander über eine kovalente Phosphodiesterverbindung verknüpft. Ligasen schließen somit Einzelstrangbrüche in Nukleinsäuredoppelsträngen unter Verknüpfung einer 5'-Phosphatgruppe einer Nukleinsäure mit der 3'-Hydroxylgruppe einer anderen Nukleinsäure.

Die erfindungsgemäße DNA-Ligase wird in Archaebakterien codiert, insbesondere in der Ordnung der Sulfolobales und im speziellen in Desulfurolobus ambivalens (Bergey's Manual, Vol. 3, S. 2250-2253 (1984), Nature Vol. 313, S. 789-791 (1985), System. Appl. Mikrobiol. 8, 197-203 (1986)). Desulfurolobus ambivalens gehört genau wie eine größere Anzahl weiterer extrem thermophiler, ungefähr zur gleichen Zeit isolierter Organismen zu dem erst kurz zuvor neu definierten Urreich der Archaebakterien (FEMS Microbiol. Rev. 75, 117-124 (1990)). Ende der siebziger Jahre hatten sich die Befunde gehäuft, daß die "Prokaryonten" nach molekularen Kriterien in zwei fundamental verschiedene Organismengruppen zerfallen. Dies veranlaßte 1977 C.R. Woese und G.E. Fox, die Prokaryonten als phylogenetische Einheit aufzutrennen, indem sie einen Teil davon als gleichberechtigtes Urreich der Archaebakterien den übrigen, den Eubakterien, gegenüberstellten und so die bis dahin als phylogenetisch bedeutsam angesehene Prokaryonten-Eukaryonten-Dichotomie durch eine Trichotomie ersetzten. Seither ist eine große Anzahl von bis dahin unbekannten Archaebakterien beschrieben worden, und auch die Erkenntnisse auf den Gebieten der Molekularbiologie und Biochemie der Archaebakterien haen das Bild der Gruppe als monophyletische Einheit des Lebens gefestigt (J. Mol. Evol. 24, 167-173 (1986)). Mit der 1990 vorgeschlagenen Neubenennung der Domänen der Eucarya, Archaea und Bacteria als höchste Taxa anstelle der Urreiche der Eukaryonten, Archaebakterien und Eubakterien wurde dieser Weiterentwicklung Rechnung getragen.

Desulfurolobus ambivalens aus der Ordnung der Sulfolobales wächst bei einer Temperatur von 55-85° C und in einem Bereich von pH 0,8-4 (Optima 80°C und pH 2,5). Es ist strikt chemolithoautotroph und kann auf einem reinen Mineralmedium unter CO₂-Assimilation durch Reduktion oder Oxidation von Schwefel wachsen.

Desulfurolobus ambivalens ist aus der öffentlichen Sammlung der DSM unter Nr. 3772 erhältlich. Aufgrund der nachfolgend gegebenen Identifikation des Enzyms kann das native Enzym nach bekannten Methoden (z.B. Proteinisolierung, Trennung nach Molekulargewicht oder Ladung und Aktivitätstest) aus Desulfurolobus ambivalens isoliert werden.

Die erfindungsgemäße Ligase ist bevorzugt zwischen 560 und 750 Aminosäuren lang. Besonders bevorzugt beträgt die Länge des erfindungsgemäßen Enzyms ca. 600 Aminosäuren.

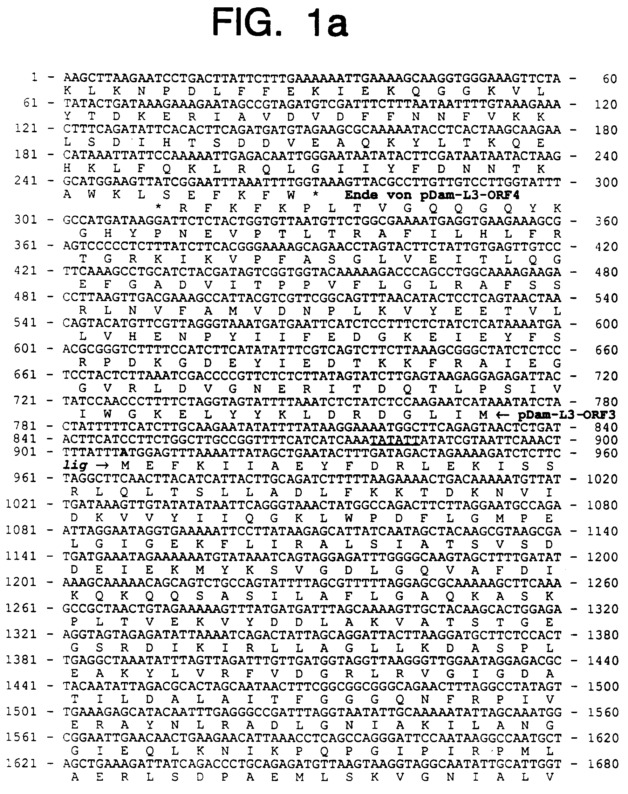

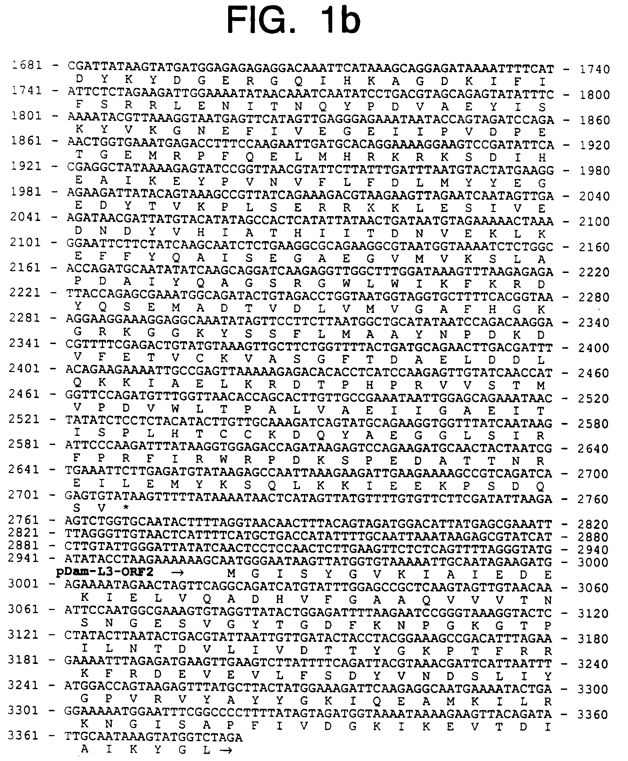

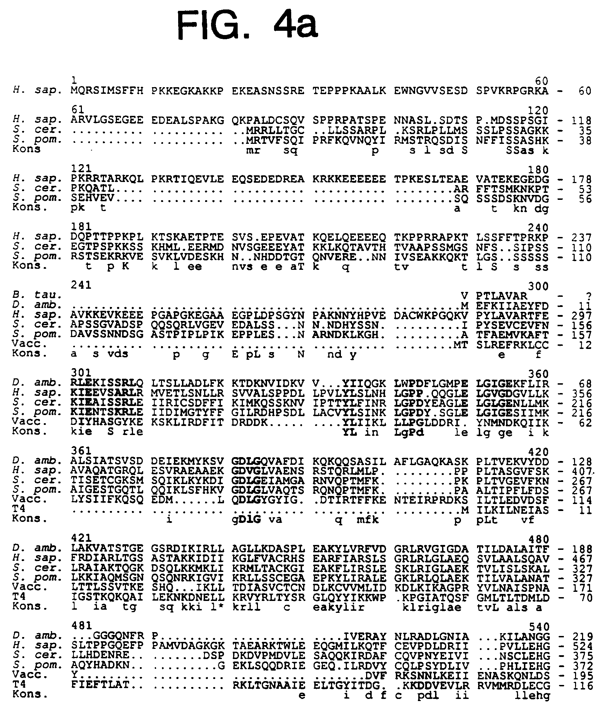

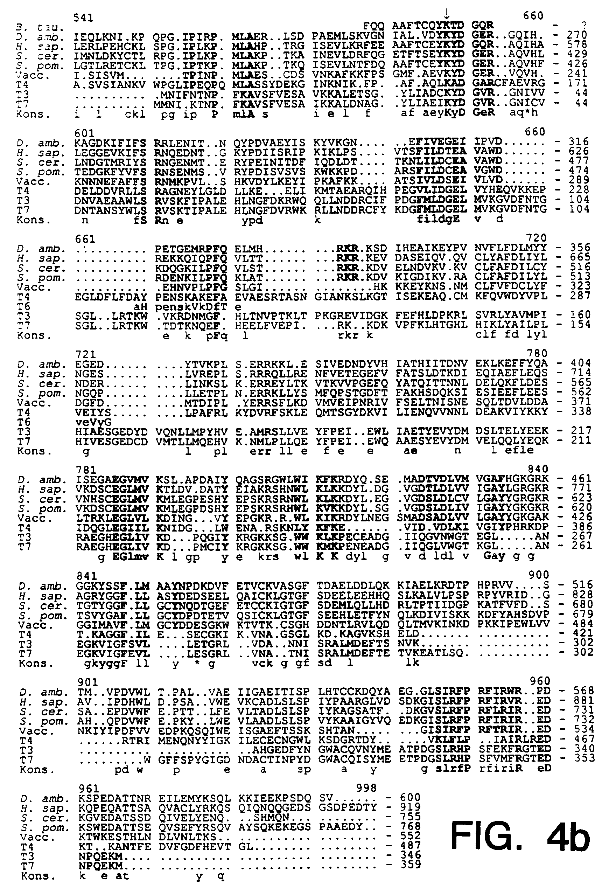

Die erfindungsgemäße Ligase ist abhängig von ATP als Cofaktor. Die Aminosäuresequenz der DNA-Ligase aus Desulfurolobus ambivalens ist in FIG 1 angegeben. Die Homologie dieser Aminosäuresequenz mit eukaryonten DNA-Ligasen beträgt nur ca. 30-35 % (FIG 4). Die Homologie zu Phagenligasen ist noch niedriger. Nennenswerte Homologien zu NAD-abhängigen Ligasen aus Eubakterien konnten (mit Ausnahme des AMP-bindenden Motivs) nicht gefunden werden. Die erfindungsgemäße DNA-Ligase enthält daher entweder die Aminosäuresequenz der FIG 1 oder einen Teil dieser Aminosäuresequenz, der mehr als 50 Aminosäuren lang ist und Ligasenaktivität aufweist, oder eine Aminosäuresequenz, die zu mindestens 40 %, bevorzugt mindestens 60, besonders bevorzugt mindestens 90 % homolog zu einer der genannten Sequenzen ist und Ligaseaktivität aufweist. Sie hat ferner bevorzugt weniger als 30 % Homologie zu der Aminosäuresequenz von eukaryonten DNA-Ligasen. Das Enzym hat bevorzugt ein Molekulargewicht zwischen 60 und 80 kD, besonders bevorzugt von ca. 67 kD. Der isoelektrische Punkt des Proteins aus D. ambivalens (aus der Aminosäuresequenz berechnet) ist 6.21. Die Gesamtladung beträgt -6. Der Gehalt an hydrophoben Aminosäuren (I + L + V + M) ist 26.2 %, was innerhalb des Bereiches anderer bekannter DNA-Ligasegene liegt (20.6 - 26.8 %).

Das erfindungsgemäße Enzym ist bei Temperaturen von über 55°C, bevorzugt zwischen 75 und 95°C, besonders bevorzugt bei etwa 80°C, stabil. Insbesondere ist das Enzym stabil unter Bedingungen, welche die mehrmalige Behandlung von Nukleinsäuren unter thermisch denaturierenden Bedingungen beinhalten. Besonders bevorzugt ist das Enzym säurestabil von pH 4 bis pH 8.5, bevorzugt im Bereich von pH 6-7.5. Das erfindungsgemäße Enzym ist aktiv mindestens in dem Intervall von 50-85°C, insbesondere zwischen 50 und 60°C.

Größere Mengen des Enzyms können auch durch rekombinante Herstellung gewonnen werden. Dazu wird eine für das Enzym kodierende Nukleinsäure in einen Vektor eingebracht und in einem geeigneten Wirt zur Expression gebracht. Die erforderlichen Arbeitsschritte für die molekulare Klonierung eines Gens sind prinzipiell aus Sambrook, Maniatis et al., Molecular Cloning: A Laboratory Manual, Cold Spring Harbour Laboratory Press (1989) bekannt. Die für die Ligase kodierenden Nukleinsäuren (DNA) können in Archaebakterien, speziell in Sulfolobales, beispielsweise mit Oligonukleotiden der SEQ ID Nrn. 1 bis 4 identifiziert werden.

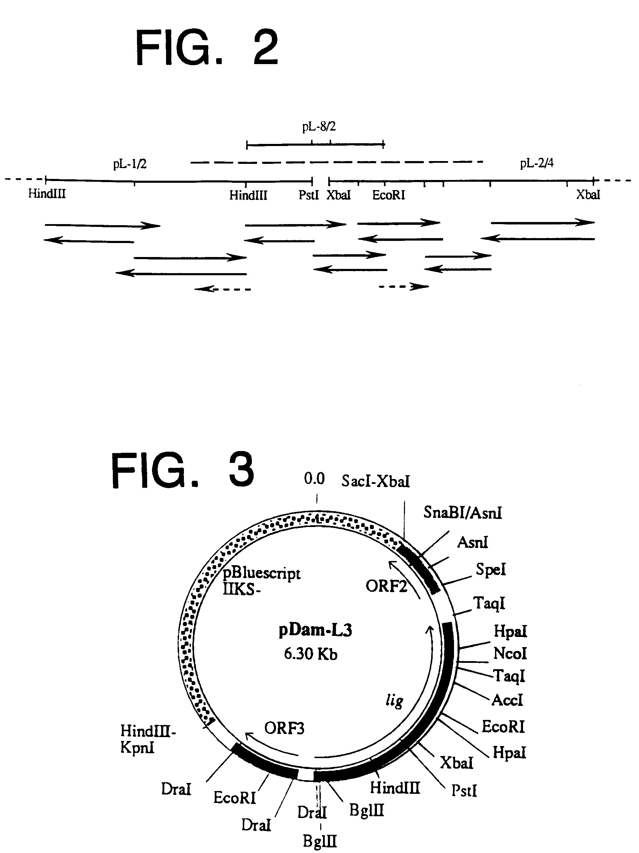

Ein Vektor, welcher eine für die erfindungsgemäße Ligase kodierende Nukleinsäure enthält, ist beispielsweise pDam-L3. Dieser Vektor wurde erhalten durch Klonierung eines 857 bp HindIII/EcoRI DNA-Fragments von D. ambivalens in pUC18 (host strain E. coli JM 83, Klon pL 8/2). Dazu wurde genomische DNA endonukleolytisch verdaut und Southern-Transfer und Hybridisierung nach Standardvorschrift (Maniatis et al., Molecular Cloning, a laboratory manual, Cold Spring Harbour Laboratory Press (1989) und anschließende Identifizierung des Fragments mit ³²P-markiertem Oligonukleotid SEQ ID 1 durchgeführt. Die Oligonukleotide SEQ ID 2 und 3 wurden benutzt um zwei verschiedene PstI/XbaI Fragmente zu identifizieren und in pUC18 (Plasmide pL1/2 und pL2/4) zu klonieren, welche den N-terminalen und den C-terminalen Teil des DNA-Ligase-Gens von Desulfurolobus ambivalens enthalten (FIG 2). Danach wurde pDam-L3 aus diesen drei Klonen durch Ligation des EcoRI/XbaI Fragments von pL 2/4 in p-BluescriptIIKS⁻ (Stratagene), gefolgt vom EcoR1-/HindIII-Fragment von pL 8/2 und dem HindIII Fragment von pL 1/2 zusammengesetzt (FIG 3).

Um das Gen in E.coli exprimieren zu können, wurde die codierende Sequenz mit Hilfe der Oligonukleotide SEQ ID NO 9 und SEQ ID NO 10 als Primer in einem PCR-Ansatz amplifiziert. Diese Primer enthalten im 5'-Bereich zusätzlich eine für das Restriktionsenzym BamHI-spezifische Sequenz. Nach BamHI-Verdau wurde das PCR-Fragment in den BamHI-gespaltenen Expressionsvektor pQE-8 kloniert (Diagen, QIAexpress Vektor Kit SRF, Literatur: The QIAexpressionist; Stüber et al., 1990, in Immunologcial Methods, eds. I. Lefkovits and B. Pernis, Vol. IV, 121-152). Das neukombinierte Plasmid wurde pQE-L6 bezeichnet. Nach Transformation in den E.coli-Stamm M15 wurde das Expressionsprodukt wie in Beispiel 8 gereinigt. Dabei enthält M15 zusätzlich das Plasmid pREP4, welches für das Neomycinphosphortransferase-Gen (neo) und das lacI-Gen (= lac-Repressor) codiert. Das neo-Gen vermittelt dem Stamm eine Kanamycin-Resistenz (zusätzlich zu der Ampicillin-Resistenz, welche über pQE-L6 eingebracht wurde). Der mit dem Plasmid pQE-L6 transformierte Stamm M 15 wurde bei DSM (Deutsche Sammlung von Mikroorganismen und Zellkulturen) unter Nr. 7070 am 19.05.1992 hinterlegt.

Die erfindungsgemäßen Nukleinsäuren hybridisieren nur mit DNA aus Sulfolobus acidocaldarius und Sulfolobus shibatae. Hybridisierung mit genomischen Sequenzen der Ligasegene von Organismen außerhalb der Archaebakterien fand auch unter relaxierten Bedingungen nicht satt. Dies bedeutet, daß das Ligase-Gen eine Nukleotidsequenz hat, die sich stark von der der bekannten Ligasegene unterscheidet und daß die Ligasegene aus Archaebakterien und insbesondere von Sulfolobacae, untereinander starke Ähnlichkeiten zeigen.

Das Plasmid pDam-L3 enthält drei offene Leserahmen, wovon die Sequenz von Basenpaar 908 bis 2710 (FIG 1) der für die Ligase kodierenden Sequenz entspricht. Die Sequenz kodiert für ein Protein von 67619 D (errechnet), welches 600 Aminosäuren enthält. Der GC-Gehalt der kodierenden Region für die erfindungsgemäße DNA-Ligase beträgt ca. 36 %, leicht oberhalb des Gesamt-GC-Gehalts von Desulfurolobus ambivalens DNA (31 %). In FIG 1 ist die Nukleotidsequenz der erfindungsgemäßen Nukleinsäure und die entsprechende Aminosäuresequenz angegeben.

Der Fachmann kann diese Nukleotidsequenz dahingehend abändern, daß sich keine Änderungen in der Aminosäuresequenz ergeben (Degenerierung des genetischen Codes). Es ist anzunehmen, daß von Änderungen bis zu einem bestimmten Grad in der Nukleotidsequenz, welche auch zu Aminosäureänderungen führen, keinen Einfluß auf die Aktivität der erfindungsgemäßen Ligase zu erwarten ist. Bevorzugt umfaßt die Erfindung somit auch Nukleinsäuren, welche zu mehr als 40 %, bevorzugt mehr als 70 %, besonders bevorzugt jedoch mehr als 90 % zu der Sequenz in FIG 1 bzw. der genannten Teilsequenzen komplementär sind. Auch in Bezug auf die Länge des Ligasegens ist anzunehmen, daß eine Abweichung von der Länge des Gens aus Desulfurolobus ambivalens in gewissem Umfang keine entscheidende Änderung der Funktion der Kodierung für die erfindungsgemäße Ligase bewirkt. Bevorzugt ist die Nukleinsäure mindestens 150 bp, insbesondere zwischen 1000 und 3000 bp, lang. Die Nukleotidsequenz enthält insbesondere ein Motiv, welches für eine ATP-Bindungsstelle der Aminosäuresequenz K_{.}DG_{.}R kodiert.

Die erfindungsgemäße Nukleinsäure kann auch dahingehend definiert werden, daß ihr komplementärer Strang unter den Bedingungen 6xSSC, 50 % Formamid, Raumtemperatur oder auch stringenter, mit nicht zum Vektor gehörigen Sequenzen und somit Desulfurolobus-spezifischen Sequenzen der Nukleinsäure aus FIG 1, insbesondere dem Teil der Nukleotide Nr. 908 bis 2716, besonders bevorzugt 988-2457, aus pDam-L3 oder mit dem 3382 bp langen XbaI/HindIII-Fragment aus Desulfurolobus ambivalens hybridisiert.

Solche Nukleinsäuren sind beispielsweise die Oligonukleotide der SEQ ID NO 1 - 4 und 7 - 10. Sie können beispielsweise als Primer zur templatabhängigen Extension (z.B. PCR) oder nach Einführung einer Markierung als Nachweissonden für Desulfurolobus ambivalens bzw. des Ligasegens eingesetzt werden.

Ebenfalls Gegenstand der Erfindung ist ein Verfahren zum Nachweis von Nukleinsäuren unter Verwendung der erfindungsgemäßen Ligase zur Verknüpfung zweier Nukleinsäuren, welche in benachbarten Bereichen mit einer nachzuweisenden Nukleinsäure hybridisiert sind.

Ein Beispiel für einen Nachweis unter Verwendung einer Ligase ist in der EP-A-0 320 308 beschrieben. Die Bedingungen für die Ligase-Chain-Reaction können aus diesem Dokument übernommen werden, wenn für die dort beschriebene Ligase die erfindungsgemäße Ligase eingesetzt wird. Die ATP-Abhängigkeit der erfindungsgemäßen Ligase muß jedoch berücksichtigt werden. Aus diesem Grund sollte ATP während der Ligasereaktion in einer Konzentration von mehr als 0.1 mmol/l, bevorzugt zwischen 0.1 und 20 mmol/l vorhanden sein. Die Konzentration von Polyethylenglykol (PEG) kann bei dem erfindungsgemäßen Verfahren 0 bis 20 % bevorzugt 5 bis 10 % betragen.

Ein weiteres Verfahren zum Nachweis von Nukleinsäuren, bei dem die erfindungsgemäße Ligase zur Ligation von benachbarten Nukleinsäuren eingesetzt werden kann, ist in WO 90/01069 beschrieben. Hierbei wird zunächst eine Lücke von wenigen Nukleotiden zwischen benachbart hybridisierten Nukleotiden durch Einbau von Mononukleosidtriphosphaten aufgefüllt und die entstandene Lücke (ein sogenannter Nick) mit der Ligase geschlossen.

In FIG 1 (FIG 1a und FIG 1b) ist die Nukleotidsequenz von pDam-L3, inklusive der vollständigen Sequenz für die DNA-Ligase aus Desulfurolobus ambivalens und pDam-L3 ORF 3 angegeben, sowie Teile der Sequenzen von pDam-L3 ORF 2 und 4. Unterstrichen: Box A; Fett: Transkriptionsstart.

In FIG 2 ist das Klonierungs- und Sequenzierungsschema für Plasmid pDam-L3 gezeigt.

Die gestrichelten Pfeile markieren Sequenzen, die mit den Oligonukleotiden L1 (SEQ ID NO 2) und L2 (SEQ ID NO 3) gewonnen wurden und zur Überprüfung der Ligationsergebnisse dienten, durchzogene Pfeile die übrigen Sequenzen, gewonnen durch Subklonierung der entsprechenden Fragmente. Die lang gestrichelte Linie markiert die Lage des DNA-Ligasegens, rechts und links gestrichelt ist die Fortsetzung der Plasmide pL-1/2 und 2/4 angezeigt.

FIG 3 enthält die Plasmidkarte von pDam-L3 mit Restriktionsschnittstellen. Ebenfalls eingezeichnet sind die Orientierungen der Leserahmen. Die 0-Marke entspricht der 0-Marke von p-Bluescript IIKS⁻. lig entspricht dem DNA-Ligasegen.

FIG 4 enthält einen Vergleich der Aminosäuresequenzen bekannter DNA-Ligasen. Auf der rechten Seite ist die Aminosäurenumerierung der individuellen Sequenz angegeben. Über der Sequenz ist die Numerierung der längsten Ligasesequenz (H. sapiens) sowie eine besonders homologe Stelle (aa 587 bis 593) angegeben. Fettgedruckt sind hochkonservierte Teile. Der Pfeil zeigt die ATP-Bindungsstelle (Lysin). Ein Eintrag in der Konsensuslinie (letzte Zeile, Kons) wurde im Fall von mehr als 50 % Identität gemacht. Ein Stern entspricht 1:1 Verteilung, Großbuchstaben bedeuten Identität über alle verglichenen Sequenzen.

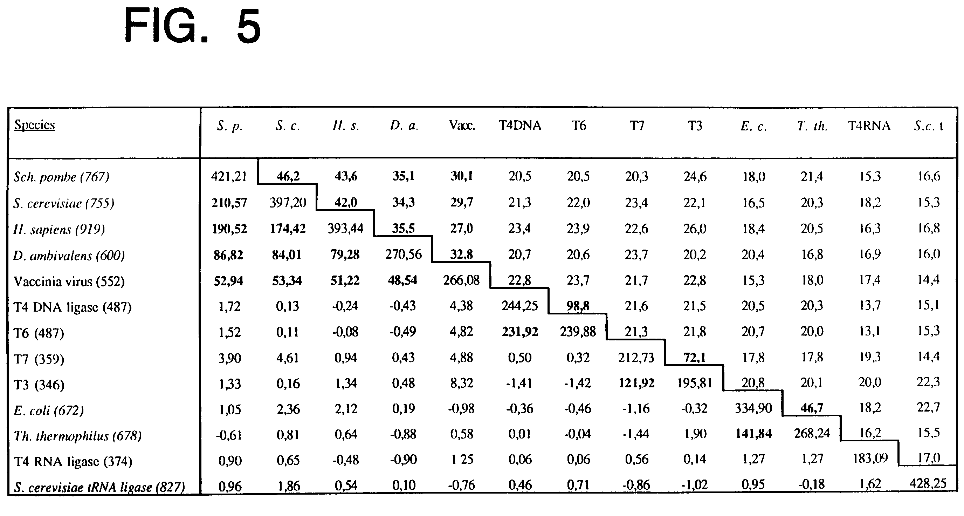

FIG 5 zeigt einen paarweisen Vergleich der bekannten DNA-Ligasesequenzen unter Einschluß der RNA-Ligase von Bacteriophagen T4 und der tRNA-Ligase von S. cerevisiae. Im oberen rechten Dreieck sind die paarweisen Identitäten (%), bestimmt mit dem Programm GAP, angegeben, im unteren linken Dreieck die Signifikanzanalyse der Sequenzhomologie mit dem Programm RDF 2. Alle Werte der RDF 2-Bestimmungen sind SD-Einheiten, daß heißt die Trefferquote der paarweisen Ausrichtung wurden durch die Standardabweichungen der zufälligen Ausrichtungen geteilt. In der mittleren Diagonale sind zur Kontrolle die RDF 2-Ergebnisse der Sequenzen mit sich selber aufgetragen. In Klammern ist die Länge der jeweiligen Sequenz in Aminosäuren angegeben. In beiden Fällen wurde die jeweils längere Sequenz auf die Länge der kürzeren Sequenz reduziert, um Zufallsübereinstimmungen auszuschließen.

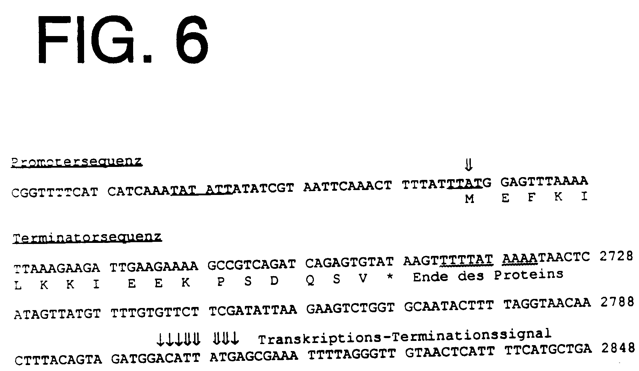

In FIG 6 sind die Ergebnisse der Transkriptionsstudien an Gesamt-RNA aus D. ambivalens gezeigt. Fette Pfeile: Hauptstart- und Terminationssignale. Unterstrichen: Box A und Box B Motive.

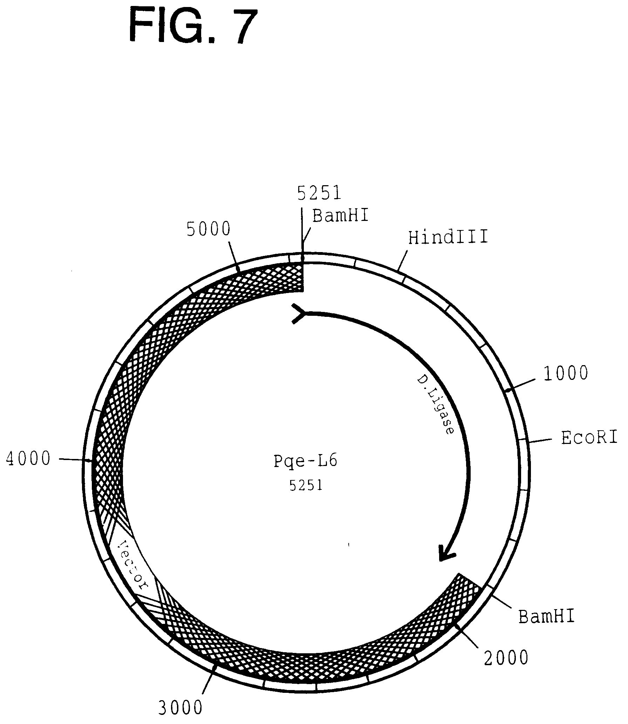

In FIG 7 ist die Plasmidkarte von pQE-L6 gezeigt. Charakteristische Restriktionsstellen für das Ligasegen sind angegeben. Die Details der Vektorkonstruktion sind zu entnehmen aus "QIA-Expressionist (DIAGEN GmbH, Düsseldorf, BRD)".

Die Erfindung wird durch die folgende Beispiele weiter erläutert:

### Beispiel 1

### Identifizierung des DNA-Ligase-Gens in Desulfurolobus ambivalens

Chromosomale DNA von D. ambivalens wurde aus anaerob gezogenen Zellen nach gängigen Methoden präpariert (Zellaufschluß mit SDS, Phenol- und Chloroformextraktionen, Ethanolfällung, zweimalige CsCl-Dichtegradientenzentrifugation (Molecular Cloning; A laboratory manual CSH Laboratory Press 1989). Gesamt-RNA aus anaerob und aerob gezogenen Zellen wurde mittels der Guanidinium-Thiocyanat-Methode präpariert (Anal. Biochem. 162, 156-159 (1987)). Die Präparation von Plasmiden erfolgte nach Angaben des Herstellers über Quiagen-Säulen geeigneter Kapazität.

Oligodesoxynukleotide SEQ ID NO 1-4 und 7-10 wurden auf einem DNA Synthetisator 381 (Applied Biosystems, Weiterstadt) synthetisiert.

Die Oligonukleotide wurden mit -³²P-ATP endmarkiert, indem sie in PNK-Puffer (J. Mol. Evol. 24, 167-173 (1986)) im Verhältnis 1 pmol Oligonukleotid zu 10 µCi mit 5 U T4-Polynukleotidkinase für 30 min bei Raumtemperatur inkubiert wurden. Die Prähybridisierung von Filtern erfolgte in 6 x SSC, 1 x Denhardt's 0,5 % SDS, 20 µg/ml tRNA (E. coli) und 0,05 % tetra-Natriumdiphosphat-10-hydrat bei 65°C für 30-120 min. Die Hybridisierung erfolgte in dem oben genannten Puffer mit dem Unterschied, daß nur 0,1 % SDS zugegeben wurde. Hybridisierungstemperatur und -zeit wurden empirisch ermittelt. In der Regel wurden die Filter nach der Hybridisierung einmal für 15 min bei Raumtemperatur in 1 x SSC/0,5 % SDS gewaschen und einmal für 30 min bei der als optimal ermittelten Hybridisierungstemperatur. Koloniehybridisierungen (Molecular Cloning loc. cit.) wurden grundsätzlich genauso behandelt.

### Beispiel 2

### Klonierung

Ein 857 bp großes HindIII/EcoRI-Fragment wurde in pUC 18 (Amersham, Braunschweig) (pL 8/2) kloniert, nachdem es durch Spaltung genomischer DNA, Southern-Transfer und Hybridisierung mit dem -³²P-markierten Oligonukleotid Soxi (SEQ ID NO 1) identifiziert worden war.

Von der Nukleinsäuresequenz von pL-8/2 wurden zwei weitere Oligonukleotide abgeleitet, mit deren Hilfe zwei PstI/XbaI-Fragmente identifiziert und kloniert wurden, die das N-terminale (pL-1/2, Oligonukleotid SEQ ID NO 2) und das C-terminale (pL-2/4, Oligonukleotid SEQ ID NO 3) Ende des Genes enthielten. Letzteres enthielt offenbar aufgrund einer Doppelligation oder partieller Spaltung noch eine interne XbaI-Schnittstelle (FIG 2). Ausgehend von den drei teilweise überlappenden Klonen wurde das Plasmid pDam-L3 zusammengesetzt, das die vollständige genetische Information für die DNA-Ligase trug. Dazu wurden das EcoRI/XbaI-Fragment von pL2/4 zunächst in pBluescript II KS⁻ (Stratagene Cloning Systems La Jolla, USA) kloniert, gefolgt von dem EcoRI/HindIII-Fragment von pL8/2 und dem HindIII-Fragment von pL1/2. Die Ergebnisse der einzelnen Klonierungsschritte wurden jeweils durch Doppelstrangsequenzierung mit den Oligonukleotiden SEQ ID NO 2, 3, 13und 14 überprüft (vgl. FIG 2).

Das resultierende Plasmid pDam-L3 enthielt ein DNA-Fragment von 3382 bp Länge mit der vollständigen Information für die DNA-Ligase von D. ambivalens und des weiteren ca. 900 bp stromauf und ca. 700 bp stromab (FIG 3).

### Beispiel 3

### Subklonierung und Sequenzierung von pDam-L3

Geeignete Restriktionsfragmente wurden in die Vektoren M13mp10, M13tg131, M13mp18, M13mp19 (Amersham, Braunschweig BRD), bzw. in die vier pBluescript-Vektoren (Stratagene Cloning Systems, La Jolla, USA) kloniert. Die Sequenzierstrategie ist in FIG 2 dargestellt. Sequenzreaktionen wurden je nach Erfordernissen mit Einzel- oder Doppelstrangmatrizen gemacht. Sequenzreaktionen mit in M13-Vektoren subklonierten Fragmenten wurden mit einem Universal-Oligonukleotid gestartet (= -40-Oligo des Sequenase Kits, United States Biochemical Corporation, Cleveland, Ohio, USA, SEQ ID NO 11), Reaktionen in pUC mit dem Universal bzw. dem reversen - 40 Oligonukleotid (für das reverse Oligonukleotid wurden die Angaben in Nucl. Acids Res. 17, 4897 (1989) berücksichtigt), diejenigen in pBluescript-Vektoren je nach Erfordernissen mit den Oligonukleotiden SEQ ID NO 13und 14. Bei letzteren wurde je nach Orientierung des Fragmentes im Vektor der Einzelstrang nach Infektion mit dem Helferphagen VPS13 (Stratagene Cloning Systems) für die Sequenzierung präpriert, oder aber das Fragment Doppelstrang-sequenziert. Zusätzlich wurden Doppelstrang-Sequenzierungen mit spezifisch synthetisierten Oligonukleotiden oder den Sucholigonukleotiden gemacht (s.o.). Für die Sequenzreaktionen wurde der Sequenase-Kit entsprechend den Anleitungen des Herstellers verwendet (United States Biochemical Corporation). Sequenzgele, gegossen mit Sequagel-Lösungen (National Diagnostics Corporation, Manville, New Jersey, USA), wurden in Makrophor-Sequenzierkammern gefahren (Pharmacia, LKB, Freiburg).

### Ergebnisse

Die vollständige Nukleotidsequenz und ihre Übersetzung in die Aminosäuresequenzen sind in FIG 1 dargestellt. Vier offene Leserahmen innerhalb des sequenzierten Bereiches wurden näher analysiert. Der längste (bp 908-2710, FIG 2) enthielt die Information für ein hypothetisches 67617 Da-Protein bestehend aus 600 Aminosäuren. Von den drei weiteren analyisierten Leserahmen begann einer bei bp 2961 und ging über das Ende der sequenzierten Region hinaus (pDam-L3-ORF 2), der zweite überspannte bp 771-250 (pDam-L3-ORF 3, vgl. FIG 3), der Anfang des dritten lag im nicht sequenzierten Bereich von pL1/2 und endete bei bp 273 (pDam-L3-ORF4).

### Beispiel 4

### Alignment mit pro-/eukaryonten DNA/RNA-Ligasen

Die erhaltenen Nukleotidsequenzen wurden mit Hilfe des Programmpaketes UWGCG, (Nucl. Acids Res. 12, 387-395 (1984)) Version 7.0 (1991) analysiert. Vergleiche von offenen Leserahmen mit in der Genbank Mipsx (Martinsried) gespeicherten Sequenzen wurden mit Hilfe des Programmes PIRFASTA (Meth. Enzymol. 183, 63-99 (1990)) angestellt. Die verfügbaren Aminosäuresequenzen der DNA-Ligasen von Schizosaccaromyces pombe (Enz. J. Biochem. 162, 659 (1987)), Saccharomyces cerevisiae (Nucl. Acids. Res. 13 8323-8337 (1985)), Escherichia coli (Mol. Gen. Genet. 204, 1-7 (1986)), Thermus thermophilus (J. Bacteriol. 173, 5047 (1991)), der menschlichen DNA-Ligase I (Proc. Natl. Acad. Sci. USA 87, 6679 (1990)), der Vaccina Virus DNA-Ligase Nucl. Acids Res. 17, 9051-9062 (1989), der Ligasen der E. coli-Bakteriophagen T3, J. Mol. Biol. 479-495 (1987), T4, Nucl. Acids Res. 11, 7145-7156 (1983), T6, Dokl. Akad. Nank SSSR 299, 737-742 (1988) und T7, J. Mol. Biol. 148, 303-330 (1981) der RNA-Ligase von T4, EMBO J. 3, 397-402 (1988) und der tRNA-Ligase von S. cerevisiae, J. Biol. Chem. 263, 3171-3176 (1988) wurden Sequenzvergleichen mit dem Programm GAP unterworfen, um die paarweisen Identitäten zu bestimmen (FIG 5). Nicht berücksichtigt wurden die Aminoacyl-tRNA-Ligasen, die eine ganz andere Funktion ausüben. Die Signifikanzanalyse der paarweisen Ausrichtungen wurde mit dem Programm RDF2 durchgeführt. Dieses Programm vergleicht die Ergebnisse von paarweisen Ausrichtungen mit den Ergebnissen einer einzugebenden (hier: 1000) Anzahl von Ausrichtungen, bei dem die eine der beiden Sequenzen in zufälliger Weise aufgebrochen und gemischt wird (= "shuffeln"), und es berechnet die Standardabweichung daraus. In FIG 5 sind die Vielfachen des Ergebnis der Ausrichtung geteilt durch die Standardabweichungen angegeben (SD-Einheiten). Es wurden Tests in beiden Richtungen durchgeführt, d.h. von zwei Sequenzen wurden beide jeweils einmal intakt gelassen und "geshuffelt" und die beiden Ergebnisse gemittelt. Da die ATP-abhängigen DNA-Ligasen am C-terminalen Ende stärker konserviert waren, wurden die jeweils längeren Sequenzen von N-Terminus her auf die gleiche Länge gekürzt.

Die sich aus den paarweisen Vergleichen ergebenden Prozentwerte der Identitäten, bzw. der Vielfachen der Standardabweichungen von paarweiser und zufälliger Ausrichtung (RDF2) der Aminosäuresequenzen der DNA-Ligasen sind in FIG 5 dargestellt. Auffallend ist hier, daß die DNA-Ligase von D. ambivalens bei allen Unterschieden mehr als 30 % Sequenzidentität zu allen vier bekannten ATP-abhängigen DNA-Ligasen der Eukarya hat und Werte zwischen dem 48- und 86-fachen der Standarbweichung in der RDF2 aufweist (FIG 5). Die RDF2-Ergebnisse der einzelnen Sequenzen, kontrollhalber gegen sich selber getestet (100 % Identität) lagen im Bereich zwischen 200 und 450 SD und waren vorwiegend von der Länge der Sequenz abhängig. Keine signifikante Ähnlichkeit wurde zu den NAD-abhängigen, bakteriellen, cytoplasmatischen DNA-Ligasen von E. coli und Th. thermophilus gefunden (<20,4 % Sequenzidentität; < 0,2 SD in der RDF2). Ebensowenig war eine Ähnlichkeit zu der ebenfalls ATP-abhängigen RNA-Ligase vom E. coli-Phagen T4 und der tRNA-Ligase von S. cerevisiae feststellbar. Für die T-Phagen-Ligasen lagen die Ergebnisse dazwischen. Die RDF2-Analyse ergab zwar keine signifikanten Resultate, die Identitätswerte lagen jedoch bei 20,2-23,7 %. Betrachtet man FIG 5, so fallen folgende Punkte auf:Die eukaryalen und die D. ambivalens-DNA-Ligasen bilden untereinander eng verwandte Gruppe. Die T4- und T6-Ligasen sind bis auf sechs Positionen identisch. Die T3- und T7-Ligasen sind mit 72 % Identität ebenfalls eng verwandt, ebenso die NAD-abhängigen DNA-Ligasen von E. coli und Thermus thermophilus (46,7 %), dieses Paar ist mit dem Rest jedoch nicht verwandt. Die RNA-Ligasen sind weder untereinander noch mit dem Rest in Beziehung zu bringen. Die vier bekannten DNA-Ligasen der T-Phagen sind von den übrigen ATP-abhängigen DNA-Ligasen deutlich getrennt. In der paarweisen Analyse liegen sie mit Identitätswerten von 20,2-26,0 % über dem Rest, in der Signifikanzanalyse weisen sie jedoch Werte um den Nullpunkt auf. Sie können aber aufgrund konservierter Signaturen in die Ausrichtung der ATP-abhängigen DNA-Ligasen mit aufgenommen werden (s.u.). Die Vaccina-DNA-Ligase weist eine Zwischenstellung auf. Sie ist den übrigen eukaryalen Enzymen nahe verwandt, erzielte aber auch Werte von 4,3-8,3 in der RDF2-Analyse mit den T-Phagen-Ligasen, sie verbindet somit die beiden Gruppen von Ligasen miteinander.

Im folgenden werden nur die ATP-abhängigen DNA-Ligasen behandelt, da nur bei ihnen eine gemeinsame Ausrichtung möglich war. Für alle anderen konnten keine gemeinsamen Strukturen im Sequenzvergleich gefunden werden, die sich von Zufallstreffern abheben. Die DNA-Ligasen unterscheiden sich in ihrer Gesamtlänge erheblich (346-919 Aminosäurereste), wobei die eukaryalen generell länger als die T-Phagen-Ligasen sind (346-487 gegenüber 552-919 Aminosäurereste). Die Ausrichtungen der Proteinsequenzen der eurkaryalen DNA-Ligasen untereinander zeigt viele konservierte Bereiche, die teilweise von Bereichen geringer Ähnlichkeit getrennt sind. Es war bereits in früheren Untersuchungen aufgefallen, daß speziell im N-terminalen Bereich die Ligasen aus den Hefen und dem Menschen wenig konserviert sind, und man hatte daraus den Schluß gezogen, daß diese Teile der Sequenzen keinerlei Beziehungen zueinander hätten. Drei hoch konservierte Strukturen wurden bei allen wiedergefunden, die z. T. schon in früheren Untersuchungen aufgefallen waren. Es handelte sich zum einen um das ATP-bindene Zentrum, das allerdings nur bei der Rinderligase I experimentell bestimmt worden war. Nahe bei der beschriebenen Konsensussequenz KYDGXR (K ist ATP-bindend, Pos. 650) wurden noch zwei wahrscheinlich ebenfalls zum Zentrum gehörende Strukturen gefunden (Pos. 560: PMLA, bzw. PFKA bei T3 und T7; Pos. 610: SR). Zum zweiten wurde die von Barker et al. (1985) aufgrund ihrer Konservierung fälschlicherweise als ATP-bindendes Zentrum vermutete Struktur bei Pos. 785-813 in der Ausrichtung wiedergefunden. Die dritte ist eine nahe dem C-Terminus liegende Struktur bei Pos. 945-960. Darüberhinaus erlaubten eine Reihe weiterer, weniger konservierter Strukturen die Ausrichtung der neun ATP-abhängigen DNA-Ligasen trotz geringer Signifikanz der Ähnlichkeiten zwischen T-Phagen und eukaryalen Ligasen. Das ATP-bindende Zentrum ist bei allen ATP-abhängigen DNA-Ligasen so hoch konserviert, daß die Zuordnung aufgrund der Sequenzhomologie zuverlässig erscheint.

### Beispiel 5

### Transkript-Analyse

Die Initiation der Transkription der offenen Leserahmen in vivo wurde mittels Primerextensionsanalyse bestimmt (Proc. Natl. Acad. Sci. USA 87, 5851 (1990)). Die in-vivo Termination des DNA-Ligase-Genes wurde durch S1-Kartierung mit einem α-³²-P-ATP markiertem NcoI/SnaBI-Fragment (bp 2458-3221) als Sonde bestimmt. Gesamt-RNA aus aerob und anaerob gewachsenen Zellen von D. ambivalens wurde für Transkriptionsanalyse des Ligasegens eingesetzt. Mit einer Ausnahme (RNA aus stationären Zellen) waren die Signale in der Primerextensionsreaktion mit Gesamt-RNA sehr schwach, es war aber aerob wie anaerob in gleicher Stärke nachweisbar. Die Transkription startete auf der ersten Base des ATG-Startcodons der Translation, bzw. auf dem vorgelagerten Thymidinrest. Von den übrigen Leserahmen von pDam-L3 wurde kein Startsignal erhalten.

Die Ergebnisse der Primerextensionsanalyse zeigten eindeutig, daß das DNA-Ligasegen in vivo transkribiert wird, obwohl die Stärke des Signals in der Regel gering und nur in einem Falle mit RNA aus stationären Zellen stark war. Die Promotersequenz wies die für die Archaea typische Struktur mit Box A und Box B auf. Über die übrigen Leserahmen des Fragmentes konnten keine Aussagen getroffen werden. Die Tatsache, daß kein mRNA-Startsignal gefunden wurde, schließt nicht aus, daß sie möglicherweise in bestimmten Fällen exprimiert werden könnten.

Die Termination der Transkription wurde durch SI-Kartierung an derselben RNA-Präparation bestimmt, die auch das starke Initiationssignal lieferte. Die Sequenz, der die Terminationssignale zugeordnet werden konnten, lautet ACATTATG, die vorgelagerte Basensequenz lautet AGTAGATGG. Die zugehörige Sequenz und die Lage des Signals sind in FIG 6 dargestellt. Die aus den Start- und Terminationssignalen abgeleitete Länge des Transkriptes betrug 1908-1914 Nukleotide.

### Beispiel 6

### Kreuzhybridisierung mit anderen Spezies

Genomische DNA aus den folgenden Organismen wurde jeweils doppelt mit BglII und Ncol verdaut, sowie zusätzlich mit dem den in Klammern angegebenen Enzymen(en): Archaea: Sulfolobus acidocaldarius (EcoRI), S. shibatae (EcoRI), Desulforococcus mucosus (EcoRI), Thermoproteus tenax (PstI), Thermococcus celer (PstI), Thermoplasma acidophilum (EcoRI), Methanothermus fervidus (EcoRI), Methanococcus vannielii (EcoRI) und Haloferax volcanei (Bam HI/PstI); Bacteria: Escherichia coli (HindIII), und Bacillus stearothremophilus (EcoRI); Eucarya: Box primigenius taurus (Rind, EcoRI), und Saccharomyces cervisiae (Bäckerhefe, EcoRI). Der Auftrennung der Ansätze auf Agarosegelen folgten ein Southern-Transfer und die Hybridisierung der Filter mit einem radioaktiv markierten BglII/Ncol-Fragment aus pDam-L3 (bp 988-2457, FIG 3), welches ca. 82 % des Genes enthält. Die Bedingungen waren: 6 x SSC, 50 % Formamid, Raumtemperatur; die Waschbedingungen waren 2 x SSC, Raumtemperatur. Mit in die Auftrennung eingeschlossen wurde genomische DNA von D. ambivalens (BglII/Ncol und EcoRI-geschnitten), sowie das ebenfalls BglII/Ncol-geschnittene Plasmid pDam-L3.

Die Ergebnisse der Hybridisierung zeigen, daß D. ambivalens offenbar nur eine Kopie dieses Genes trägt. Außerdem konnten definierte Banden in der DNA von Sulfolobus acidocaldarius und S. shibatae nachgewiesen werden, zwei Spezies, die innerhalb der Ordnung der Sulfolobales die am fernsten verwandten Organismen sind. Bei allen anderen Organismen konnte keine Hybridisierung erzielt werden. Aus diesen Ergebnissen läßt sich ableiten, daß innerhalb der Sulfolobales offenbar das Gen hoch konserviert ist. Darüber hinaus konnte mit dieser Methode auch keine enge Verwandtschaft zu den eukaryalen Enzymen auf DNA-Ebene festgestellt werden.

Zum jetzigen Zeitpunkt umfaßt die Ordnung der Sulfolobales die folgenden Spezies:
Sulfolobus acidocaldarius
S. solfataricus
S. shibatae
S. thuringiensis
S. metallicus
Acidianus infernus
A. brierleyi
Desulfurolobus ambivalens
Stygiolobus azoricus
Metallosphaera sedula
Weitere Neuisolate sind zu erwarten. Alle diese Spezies haben eine vergleichbare Morphologie und Lebensweise und sind auch phylogenetisch verwandt.

### Beispiel 7

### Expressionsklonierung

Konstruktion des Expressionsplasmids pQE-L6: Für die Expression des DNA-Ligase-Genes in E. coli wurde aus einer Präparation genomischer DNA mit Hilfe der Polymerase-Kettenreaktion der kodierende Bereich des Genes amplifiziert. Die Oligonukleotide SEQ ID NO 9 und 10 wurden so gestaltet, daß N-terminal eine BamHI-Schnittstelle gefolgt von einer Schnittstelle der Restriktionsprotease Faktor Xa eingefügt worden war und am C-Terminus eine BamHI-Schnittstelle. Die Amplifikationsbedingungen waren:
1 x Reaktionspuffer (Boehringer Mannheim, Kat. Nr. 1146165)
je 100 µM dNTP
2,5 U Taq-DNA-Polymerase
je 40 pmol Oligos
500 ng genom. DNA

| | |
|---|---|
| Denaturierung: | 94°C, 60 s |
| Anlagerung: | 51°C, 120 s |
| Polymerisation: | 72°C, 120 s |
| 40 Zyklen | |

Das Reaktionsprodukt wurde nach Spaltung mit BamHI in den ebenfalls mit BamHI geschnittenen und mit alkalischer Phosphatase dephosphorylierten Vektor Quia Express pQE-8 (DIAGEN GmbH, Düsseldorf, BRD) ligiert.

Expression in E. coli: Nach Transformation von E. coli wurde eine Vorkultur über Nacht auf Kanamycin- und Ampicillin-haltigem 2 x TY-Medium angezogen. Die daraus angeimpfte Hauptkultur im gleichen Medium wurde bis zu einer Zelldichte von OD₆₀₀ = 0,8-1,0 gezogen und dann mit einer IPTG-Konzentration von 2 mM induziert. Der zeitliche Verlauf der Proteininduktion wurde über SDS-Gele verfolgt; in der Regel wurden die Zellen 3 h nach Inkubation geerntet. Die Zellen wurden nach Vorbehandlung mit Lysozym aufgeschlossen, indem sie dreimal in flüssigem Stickstoff eingefroren und wieder aufgetaut wurden. Die Hitzefällung von E. coli-Proteinen erfolgte durch fünfzehnminütige Inkubatin bei 75°C und anschliessender Zentrifugation für 2 min in der Eppendorfzentrifuge.

Die PC-Reaktion mit Oligonukleotiden, die eine Amplifikation des DNA-Ligasegenes unter Einfügung einer Faktor Xa-Schnittstelle im Protein erlaubten (IEGR), ergab eine einzige scharfe Bande von ca. 1900 bp im Agarosegel. Sie wurde in pQE-8 kloniert, so daß das Oligopeptid der Sequenz MRGSHHHHHHGSIEGR N-terminal mit der DNA-Ligase fusioniert wurde. Die [6 x His]-Sequenz soll dabei eine Affinitäts-Reinigung des Fusionsproteins über Nitrilotriessigsäure-cheliertes Nickel ermöglichen. Identifizierung durch Koloniehybridisierung wurden fünf verschiedene positive Klone angezogen, die erhaltenen Plasmide durch Restriktionsspaltung auf das Vorhandensein eines Fragmentes der erwarteten Größe untersucht und der Verlauf der Expression nach IPTG-Induktion im SDS-Gel verfolgt. Vier von den fünf Klonen zeigten eine zusätzliche Proteinbande mit einem apparenten Molekulargewicht von 65000 im SDS-Gel. Das erwartete Molekulargewicht des Fusionsproteins betrug 69487. Durch eine Hitzebehandlung von Rohlysaten der induzierten Zellen konnte ein großer Teil des Zellproteins abgetrennt werden, während das induzierte Protein löslich blieb. Bei dem fünften Klon wie auch bei der Vektorkontrolle fehlte die Bande. Es wurde vermutet, daß der fünfte, in der Hybridisierung positive Klon das amplifizierte Fragment in inverser Richtung enthielt.

### Beispiel 8

### Reinigung der D. amibvalens-Ligase

E. coli-Bakterien, welche das Plasmid pQE-L6 enthalten, z.B. M15-pQE-L6, werden in 2 l DYT-Medium (J. Sambrook et al., Molecular Cloning, 1989, CSH, Vol. 3, Seite A.3) mit 20 µg/ml Ampicillin und 20 µg/ml Kanamycin bei 37°C angezüchtet. Bei OD₅₅₀ = 0.8 wird mit 1 mM IPTG induziert und nach 3 Std.- bis Übernacht-Inkubation bei 37°C geerntet. Die Bakterienzellen werden bei 6000 rpm in 500 ml - Bechern im GSA-Rotor einer Sorvall-Zentrifuge pelletiert und in 20 ml Tris, 20 mM, pH 7.6; EDTA, 1 mM und β-Mercaptoäthanol, 10 mM resuspendiert. Die Bakterienzellen werden mit Ultraschall-Behandlung aufgeschlossen. Dazu werden 4 x 5 ml Aliqouts 5 x 1 Minute bei 50 % der Ausgangsleistung eines Branson Sonifier 450 sonifiziert. Danach wird die Suspension bei 39.000 g in einem SS34-Rotor in einer Sorvall-Zentrifuge zentrifugiert und der Überstand mit 2 M KCl auf eine Salz-Endkonzentration von 300 mM gebracht.

Um die bakterielle DNA aus dem Ansatz zu reinigen, wird die Lösung auf eine DEAE-Säule (5 ml, equilibriert mit 300 mM KCl) gebracht und mit 70 ml 300 mM KCl eluiert. Der Säulendurchfluß wird 20'bei 65°C behandelt, um die E. coli Proteine zu denaturieren. Anschließend wird im SS34-Rotor bei 18500 rpm 15 Minuten zentrifugiert und der Überstand mit gesättigtem Ammoniumsulfat (Volumenverhältnis 1 : 1) gefällt. Anschließend wird erneut bei 18000 rpm zentrifugiert und das Pellet, welches die Ligase aus D. sulfurolobus enthält, in 4 ml H₂O aufgenommen.

### Beispiel 9

### Ligationsaktivität der Desulfurolobus ambivalens - Ligase

Als Target für diesen Aktivitätsnachweis wird genomische DNA des Phagen mit den Restriktionsenzymen SmaI (blunt end) und SalI (2 Nukleotide 5'-überstehende Enden) geschnitten. Bei hoher Reaktionstemperatur sind Hybride dieser Enden nicht stabil, so daß nur die beiden Endfragmente des Genoms, welche kohäsive 5'-überstehende Enden von 12 nt besitzen (sog. cos sites) als Hybrid vorliegen. Dabei liegen in diesem Hybrid zwei sogenannte "nicks" in 12 nt Abstand vor. An diesem Template kann demnach die Aktivität einer Ligase an genickter DNA getestet werden: als Produkt entsteht ein Fragment aus den beiden eine cos-site enthaltenden genomischen Endfragmenten.

3 µl der im Beispiel 8 beschriebenen Desulfurolobus ambivalens Ligase-Präparation werden in einem Gesamtvolumen von 50 µl in Tris HCl, 20 mM, pH 7.5; MgCl₂, 10 mM; Dithiothreitol (DTT), 10 mM; ATP, 1 mM; Polyethylenglycol (PEG), 5% bei 56°C zwischen 1 Stunde und 12 Stunden inkubiert. Die Reaktionsprodukte werden in einem 1 % Agarosegel aufgetrennt und mit Ethidiumbromid-Färbung sichtbar gemacht.

Vorgeschlagene Variationsbreiten für Ligation:

| | |
|---|---|
| Volumen: | 10 - 200 µl |
| Puffer: | 10 - 50 mM |
| MgCl₂: | 2 - 20 mM |
| DTT: | 0 - 20 mM |
| ATP: | 0.1 mM - 20 mM |
| PEG: | 0 - 20% |
| Temperatur: | Raumtemperatur bis 85°C, Reaktionszeit 10 Min. bis Übernacht-Inkubation. |

Wird zu der beschriebenen Ligasereaktion zusätzlich Adenosin-5'-0-(3-thiotriphosphat) (ATP-Gamma-S) 10 mM bzw. 100 mM zugegeben, ist keine Ligation der beiden Lambda-Endfragmente zu sehen. Dies weist auf eine deutliche Kompetition des Co-Faktors ATP hin und beweist die ATP-Abhängigkeit der hier verwendeten Ligase.

### Beispiel 10

### Vergleichende Cofaktorbestimmung

Im vorliegenden Beispiel wird ein Versuch beschrieben, mit welchem die ATP-Abhängigkeit der erfindungsgemäßen Ligase und die Unabhängigkeit von NAD ermittelt wurde. Zum Vergleich wurden die Cofaktoren von aus dem Stand der Technik bekannten Ligasen (T4-Ligase, Firma: Boehringer Mannheim GmbH) und Thermus spec.-Ligase (Ampligase, Firma: Biozym) ermittelt.

Zur Cofaktorbestimmung diente die Adenylierungsreaktion. Hierbei wird radioaktiv markierter Cofaktor (³²PATP oder ³²PNAD) in einem Ligationspuffer mit der Ligase inkubiert. Dadurch wird die Radioaktivität durch die Phosphorylierung der Ligase (kovalent) auf das Protein übertragen. Nach Elektrophorese in einem SDS-Gel ist die Ligase durch Autoradiographie sichtbar.

Verwendet wurden der T4-Ligase-Puffer sowie der Ampligase-Puffer.

Die Inkubationszeit betrug jeweils 1 Stunde.

Die Inkubationstemperatur betrug für die T4-Ligase 37 °C, für die Ampligase 65 °C und für die Desulforulobus Ligase 45 °C und 65 °C. Verwendet wurden von der T4-Ligase 1 U, von der Ampligase 100 U und von der Desulforulobus-Ligase 10 bis 1000 ng pro Ansatz. Das Ansatzvolumen betrug 20 µl. Bei höheren Temperaturen wurden die Ansätze mit Öl überschichtet.

T4-Ligase-Puffer: 66 mM Tris/HCl, 5 mM MgCl₂, 1 mM DTT, pH 7,5 bei 20 °C
Ampligase-Puffer: 20 mM Tris/HCl, 50 MM KCl, 10 mM MgCl₂, 1 mM EDTA, 10 mM DTT
pH 7,6 bei 65 °C
Radioaktivität: Amersham
³²PATP, spez. Aktivität: 15 TBq/mmol, 0,37 MBq Pro Ansatz
³²PNAD, spez. Aktivität: 37 TBq/mmol, 0,37 MBq pro Ansatz
Die Ampligase zeigte wie erwartet die NAD-Abhängigkeit. Die Desulfurolobus-Ligase wird eindeutig durch ATP adenyliert. Sie zeigt auch ein schwaches Signal bei NAD als Cofaktor. Dies ist aber auf eine Verunreinigung des NAD mit ATP zurückzuführen, da auch die T4-Ligase, als eindeutig ATP-abhängiges Enzym, mit NAD ein schwaches Signal erzeugt.

Der vorstehende Versuch zeigt eindeutig, daß die erfindungsgemäße Ligase im Gegensatz zu der aus einem thermophilen Eubakterium bekannten Ligase ATP-abhängig ist.

## Patentansprüche

1. Thermostabile Ligase erhältlich aus Bakterien der Gruppe der Archaebakterien.

2. Ligase gemäß Anspruch 1, erhältlich aus Bakterien der Ordnung der Sulfolobales.

3. Ligase enthaltend
- die Aminosäuresequenz der FIG 1 oder
- einen Teil der Aminosäuresequenz der FIG 1 mit mehr als 50 Aminosäuren Länge, der Ligaseaktivität aufweist, oder
- eine Aminosäuresequenz, die mindestens zu 40 % homolog zu einer der genannten Sequenzen ist und Ligaseaktivität aufweist.

4. Ligase gemäß Anspruch 1, dadurch gekennzeichnet, daß sie ATP-abhängig ist.

5. Ligase gemäß Anspruch 1, dadurch gekennzeichnet, daß sie weniger als 36 % Homologie zu der Aminosäuresequenz von eukaryonten DNA-Ligasen aufweist.

6. Ligase gemäß Anspruch 3, mit einem Molekulargewicht von ca. 67.6 kD.

7. Ligase gemäß einem der vorangehenden Ansprüche, dadurch gekennzeichnet, daß sie rekombinant hergestellt ist.

8. Ligase gemäß Anspruch 2, erhältlich aus Desulfurolobus ambivalens.

9. Thermostabile DNA-Ligase, erhältlich aus dem Expressionstamm mit der Hinterlegungsnummer DSM 7070.

10. Nukleinsäuresequenz, kodierend für eine thermostabile Ligase aus Archaebakterien.

11. Nukleinsäuresequenz nach Anspruch 10, enthaltend
- die Nukleotidsequenz der FIG 1 oder
- einen Teil davon, der für eine Ligase kodiert und mindestens 150 bp lang ist, oder
- eine Sequenz, die mindestens zu 40 % homolog zu einer der genannten Sequenzen ist und für eine Ligase kodiert.

12. Nukleinsäuresequenz, deren Komplement unter stringenten Bedingungen mit der Nukleinsäure nach FIG 1 hybridisiert.

13. Nukleinsäuresequenz nach Anspruch 12, deren Komplement unter stringenten Bedingungen mit dem 3382 bp langen XbaI/Hind III-Fragment aus Desulfurolobus ambivalens hybridisiert.

14. Nukleotidsequenz von mindestens 150 Nukleotiden Länge, enthalten in oder komplementär zu dem 1800 bp langen BamHI-Fragment aus DSM 7070.

15. Verfahren zum Nachweis einer Nukleinsäure durch Hybridisierung zweier Oligonukleotide mit benachbarten Sequenzen der Nukleinsäure, Ligation und Nachweis des Ligationsproduktes, dadurch gekennzeichnet, daß die Ligation von der Ligase gemäß Anspruch 1 mit ATP als Cofaktor vorgenommen wird.

16. Expressionsclon M15-pQE-L6 mit der Hinterlegungsnummer DSM 7070.


SEQUENZPROTOKOLL

(1) ALLGEMEINE INFORMATION:

(i) ANMELDER:
(A) NAME: Boehringer Mannheim GmbH
(B) STRASSE: Sandhoferstr. 116
(C) ORT: Mannheim
(E) LAND: DE
(F) POSTLEITZAHL: 6800
(G) TELEPHON: 0621 759 4348

(ii) ANMELDETITEL: Thermostabile Ligase aus Archaebakterien

(iii) ANZAHL DER SEQUENZEN: 14

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(vi) FRÜHERE ANMELDEDATEN:
(A) ANMELDENUMMER: DE 42 17 134.2
(B) ANMELDEDATUM: 23-MAY-1992

(2) INFORMATION ZU SEQ ID NO: 1:

(i) SEQUENZ CHARAKTERISTIKA:
(A) LÄNGE: 36 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 1:

TATGTNGCNA TWAATATGGC NGAATTRAAA AAYGAA 36

(2) INFORMATION ZU SEQ ID NO: 2:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 2:

TCCAGTGCTT GTAGCAACTT 20

(2) INFORMATION ZU SEQ ID NO: 3:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 3:

GATTATGTAC ATATAGCCAC 20

(2) INFORMATION ZU SEQ ID NO: 4:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 4:

TAAGTTGAAG CCTAGAA 17

(2) INFORMATION ZU SEQ ID NO: 5:

(i) SEQUENZ CHARAKTERISTIKA:
(A) LÄNGE: 3382 Basenpaare
(B) ART: Nukleinsäure
(C) STRANGFORM: Doppel
(D) TOPOLOGIE: linear

(ii) ART DES MOLEKÜLS: cDNS

(ix) MERKMALE:
(A) NAME/SCHLÜSSEL: promoter
(B) LAGE: 878..883

(ix) MERKMALE:
(A) NAME/SCHLÜSSEL: CDS
(B) LAGE: 908..2707

(ix) MERKMALE:
(A) NAME/SCHLÜSSEL: terminator
(B) LAGE: 2713..2722
(D) SONSTIGE ANGABEN: /note= "potentiell"

(ix) MERKMALE:
(A) NAME/SCHLÜSSEL: terminator
(B) LAGE: 2804..2811
(D) SONSTIGE ANGABEN: /note= "experimentell"

(xi) SEQUENZBESCHREIBUNG: SEQ ID NO: 5:

AAGCTTAAGA ATCCTGACTT ATTCTTTGAA AAAATTGAAA AGCAAGGTGG GAAAGTTCTA 60

TATACTGATA AAGAAAGAAT AGCCGTAGAT GTCGATTTCT TTAATAATTT TGTAAAGAAA 120

CTTTCAGATA TTCACACTTC AGATGATGTA GAAGCGCAAA AATACCTCAC TAAGCAAGAA 180

CATAAATTAT TCCAAAAATT GAGACAATTG GGAATAATAT ACTTCGATAA TAATACTAAG 240

GCATGGAAGT TATCGGAATT TAAATTTTGG TAAAGTTACG CCTTGTTGTC CTTGGTATTT 300

GCCATGATAA GGATTCTCTA CTGGTGTTAA TGTTCTGGCG AAAATGAGGT GAAGAAAGCG 360

AGTCCCCCTC TTTATCTTCA CGGGAAAAGC AGAACCTAGT ACTTCTATTG TGAGTTGTCC 420

TTCAAAGCCT GCATCTACGA TAGTCGGTGG TACAAAAAGA CCCAGCCTGG CAAAAGAAGA 480

CCTTAAGTTG ACGAAAGCCA TTACGTCGTT CGGCAGTTTA ACATACTCCT CAGTAACTAA 540

CAGTACATGT TCGTTAGGGT AAATGATGAA TTCATCTCCT TTCTCTATCT CATAAAATGA 600

ACGCGGGTCT TTTCCATCTT CATATATTTC GTCAGTCTTC TTAAAGCGGG CTATCTCTCC 660

TCCTACTCTT AAATCGACCC CGTTCTCTCT TATAGTATCT TGAGTAAGAG GAGAGATTAC 720

TATCCAACCC TTTTCTAGGT AGTATTTTAA ATCTCTATCT CCAAGAATCA TAAATATCTA 780

CTATTTTTCA TCTTGCAAGA ATATATTTTA TAAGGAAAAT GGCTTCAGAG TAACTCTGAT 840

ACTTCATCCT TCTGGCTTGC CGGTTTTCAT CATCAAATAT ATTATATCGT AATTCAAACT 900

TTTATTT ATG GAG TTT AAA ATT ATA GCT GAA TAC TTT GAT AGA CTA GAA 949
Met Glu Phe Lys Ile Ile Ala Glu Tyr Phe Asp Arg Leu Glu
1               5                   10

AAG ATC TCT TCT AGG CTT CAA CTT ACA TCA TTA CTT GCA GAT CTT TTT 997
Lys Ile Ser Ser Arg Leu Gln Leu Thr Ser Leu Leu Ala Asp Leu Phe
15                  20                  25                  30

AAG AAA ACT GAC AAA AAT GTT ATT GAT AAA GTT GTA TAT ATA ATT CAG 1045
Lys Lys Thr Asp Lys Asn Val Ile Asp Lys Val Val Tyr Ile Ile Gln
                35                  40                  45

GGT AAA CTA TGG CCA GAC TTC TTA GGA ATG CCA GAA TTA GGA ATA GGT 1093
Gly Lys Leu Trp Pro Asp Phe Leu Gly Met Pro Glu Leu Gly Ile Gly
            50                  55                  60

GAA AAA TTC CTT ATA AGA GCA TTA TCA ATA GCT ACA AGC GTA AGC GAT 1141
Glu Lys Phe Leu Ile Arg Ala Leu Ser Ile Ala Thr Ser Val Ser Asp
        65                  70                  75

GAT GAA ATA GAA AAA ATG TAT AAA TCA GTA GGA GAT TTG GGG CAA GTA 1189
Asp Glu Ile Glu Lys Met Tyr Lys Ser Val Gly Asp Leu Gly Gln Val
    80                  85                  90

GCT TTT GAT ATA AAG CAA AAA CAG CAG TCT GCC AGT ATT TTA GCG TTT 1237
Ala Phe Asp Ile Lys Gln Lys Gln Gln Ser Ala Ser Ile Leu Ala Phe
95                  100                 105                 110

TTA GGA GCG CAA AAA GCT TCA AAG CCG CTA ACT GTA GAA AAA GTT TAT 1285
Leu Gly Ala Gln Lys Ala Ser Lys Pro Leu Thr Val Glu Lys Val Tyr
                115                 120                 125

GAT GAT TTA GCA AAA GTT GCT ACA AGC ACT GGA GAA GGT AGT AGA GAT 1333
Asp Asp Leu Ala Lys Val Ala Thr Ser Thr Gly Glu Gly Ser Arg Asp
            130                 135                 140

ATT AAA ATC AGA CTA TTA GCA GGA TTA CTT AAG GAT GCT TCT CCA CTT 1381
Ile Lys Ile Arg Leu Leu Ala Gly Leu Leu Lys Asp Ala Ser Pro Leu
        145                 150                 155

GAG GCT AAA TAT TTA GTT AGA TTT GTT GAT GGT AGG TTA AGG GTT GGA 1429
Glu Ala Lys Tyr Leu Val Arg Phe Val Asp Gly Arg Leu Arg Val Gly
    160                 165                 170

ATA GGA GAC GCT ACA ATA TTA GAC GCA CTA GCA ATA ACT TTC GGC GGC 1477
Ile Gly Asp Ala Thr Ile Leu Asp Ala Leu Ala Ile Thr Phe Gly Gly
175                 180                 185                 190

GGG CAG AAC TTT AGG CCT ATA GTT GAA AGA GCA TAC AAT TTG AGG GCC 1525
Gly Gln Asn Phe Arg Pro Ile Val Glu Arg Ala Tyr Asn Leu Arg Ala
                195                 200                 205

GAT TTA GGT AAT ATT GCA AAA ATA TTA GCA AAT GGC GGA ATT GAA CAA 1573
Asp Leu Gly Asn Ile Ala Lys Ile Leu Ala Asn Gly Gly Ile Glu Gln
            210                 215                 220

CTG AAG AAC ATT AAA CCT CAG CCA GGG ATT CCA ATA AGG CCA ATG CTA 1621
Leu Lys Asn Ile Lys Pro Gln Pro Gly Ile Pro Ile Arg Pro Met Leu
        225                 230                 235

GCT GAA AGA TTA TCA GAC CCT GCA GAG ATG TTA AGT AAG GTA GGC AAT 1669
Ala Glu Arg Leu Ser Asp Pro Ala Glu Met Leu Ser Lys Val Gly Asn
    240                 245                 250

ATT GCA TTG GTC GAT TAT AAG TAT GAT GGA GAG AGA GGA CAA ATT CAT 1717
Ile Ala Leu Val Asp Tyr Lys Tyr Asp Gly Glu Arg Gly Gln Ile His
255                 260                 265                 270

AAA GCA GGA GAT AAA ATT TTC ATA TTC TCT AGA AGA TTG GAA AAT ATA 1765
Lys Ala Gly Asp Lys Ile Phe Ile Phe Ser Arg Arg Leu Glu Asn Ile
                275                 280                 285

ACA AAT CAA TAT CCT GAC GTA GCA GAG TAT ATT TCA AAA TAC GTT AAA 1813
Thr Asn Gln Tyr Pro Asp Val Ala Glu Tyr Ile Ser Lys Tyr Val Lys
            290                 295                 300

GGT AAT GAG TTC ATA GTT GAG GGA GAA ATA ATA CCA GTA GAT CCA GAA 1861
Gly Asn Glu Phe Ile Val Glu Gly Glu Ile Ile Pro Val Asp Pro Glu
        305                 310                 315

ACT GGT GAA ATG AGA CCT TTC CAA GAA TTG ATG CAC AGG AAA AGG AAG 1909
Thr Gly Glu Met Arg Pro Phe Gln Glu Leu Met His Arg Lys Arg Lys
    320                 325                 330

TCC GAT ATT CAC GAG GCT ATA AAA GAG TAT CCG GTT AAC GTA TTC TTA 1957
Ser Asp Ile His Glu Ala Ile Lys Glu Tyr Pro Val Asn Val Phe Leu
335                 340                 345                 350

TTT GAT TTA ATG TAC TAT GAA GGA GAA GAT TAT ACA GTA AAG CCG TTA 2005
Phe Asp Leu Met Tyr Tyr Glu Gly Glu Asp Tyr Thr Val Lys Pro Leu
                355                 360                 365

TCA GAA AGA CGT AAG AAG TTA GAA TCA ATA GTT GAA GAT AAC GAT TAT 2053
Ser Glu Arg Arg Lys Lys Leu Glu Ser Ile Val Glu Asp Asn Asp Tyr
            370                 375                 380

GTA CAT ATA GCC ACT CAT ATT ATA ACT GAT AAT GTA GAA AAA CTA AAG 2101
Val His Ile Ala Thr His Ile Ile Thr Asp Asn Val Glu Lys Leu Lys
        385                 390                 395

GAA TTC TTC TAT CAA GCA ATC TCT GAA GGC GCA GAA GGC GTA ATG GTA 2149
Glu Phe Phe Tyr Gln Ala Ile Ser Glu Gly Ala Glu Gly Val Met Val
    400                 405                 410

AAA TCT CTG GCA CCA GAT GCA ATA TAT CAA GCA GGA TCA AGA GGT TGG 2197
Lys Ser Leu Ala Pro Asp Ala Ile Tyr Gln Ala Gly Ser Arg Gly Trp
415                 420                 425                 430

CTT TGG ATA AAG TTT AAG AGA GAT TAC CAG AGC GAA ATG GCA GAT ACT 2245
Leu Trp Ile Lys Phe Lys Arg Asp Tyr Gln Ser Glu Met Ala Asp Thr
                435                 440                 445

GTA GAC CTG GTA ATG GTA GGT GCT TTT CAC GGT AAA GGA AGG AAA GGA 2293
Val Asp Leu Val Met Val Gly Ala Phe His Gly Lys Gly Arg Lys Gly
            450                 455                 460

GGC AAA TAT AGT TCC TTC TTA ATG GCT GCA TAT AAT CCA GAC AAG GAC 2341
Gly Lys Tyr Ser Ser Phe Leu Met Ala Ala Tyr Asn Pro Asp Lys Asp
        465                 470                 475

GTT TTC GAG ACT GTA TGT AAA GTT GCT TCT GGT TTT ACT GAT GCA GAA 2389
Val Phe Glu Thr Val Cys Lys Val Ala Ser Gly Phe Thr Asp Ala Glu
    480                 485                 490

CTT GAC GAT TTA CAG AAG AAA ATT GCC GAG TTA AAA AGA GAC ACA CCT 2437
Leu Asp Asp Leu Gln Lys Lys Ile Ala Glu Leu Lys Arg Asp Thr Pro
495                 500                 505                 510

CAT CCA AGA GTT GTA TCA ACC ATG GTT CCA GAT GTT TGG TTA ACA CCA 2485
His Pro Arg Val Val Ser Thr Met Val Pro Asp Val Trp Leu Thr Pro
                515                 520                 525

GCA CTT GTT GCC GAA ATA ATT GGA GCA GAA ATA ACT ATA TCT CCT CTA 2533
Ala Leu Val Ala Glu Ile Ile Gly Ala Glu Ile Thr Ile Ser Pro Leu
            530                 535                 540

CAT ACT TGT TGC AAA GAT CAG TAT GCA GAA GGT GGT TTA TCA ATA AGA 2581
His Thr Cys Cys Lys Asp Gln Tyr Ala Glu Gly Gly Leu Ser Ile Arg
        545                 550                 555

TTC CCA AGA TTT ATA AGG TGG AGA CCA GAT AAG AGT CCA GAA GAT GCA 2629
Phe Pro Arg Phe Ile Arg Trp Arg Pro Asp Lys Ser Pro Glu Asp Ala
    560                 565                 570

ACT ACT AAT CGT GAA ATT CTT GAG ATG TAT AAG AGC CAA TTA AAG AAG 2677
Thr Thr Asn Arg Glu Ile Leu Glu Met Tyr Lys Ser Gln Leu Lys Lys
575                 580                 585                 590

ATT GAA GAA AAG CCG TCA GAT CAG AGT GTA TAAGTTTTTA TAAAATAACT 2727
Ile Glu Glu Lys Pro Ser Asp Gln Ser Val
                595                 600

CATAGTTATG TTTTGTGTTC TTCGATATTA AGAAGTCTGG TGCAATACTT TTAGGTAACA 2787

ACTTTACAGT AGATGGACAT TATGAGCGAA ATTTTAGGGT TGTAACTCAT TTTCATGCTG 2847

ACCATATTTT GCAATTAAAT AAGAGCGTAT CATCTTGTAT TGGGATTATA TCAACTCCTC 2907

CAACTCTTGA AGTTCTCTCA GTTTTAGGGT ATGATATACC TAAGAAAAAA GCAATGGGAA 2967

TAAGTTATGG TGTAAAAATT GCAATAGAAG ATGAGAAAAT AGAACTAGTT CAGGCAGATC 3027

ATGTATTTGG AGCCGCTCAA GTAGTTGTAA CAAATTCCAA TGGCGAAAGT GTAGGTTATA 3087

CTGGAGATTT TAAGAATCCG GGTAAAGGTA CTCCTATACT TAATACTGAC GTATTAATTG 3147

TTGATACTAC CTACGGAAAG CCGACATTTA GAAGAAAATT TAGAGATGAA GTTGAAGTCT 3207

TATTTTCAGA TTACGTAAAC GATTCATTAA TTTATGGACC AGTAAGAGTT TATGCTTACT 3267

ATGGAAAGAT TCAAGAGGCA ATGAAAATAC TGAGGAAAAA TGGAATTTCG GCCCCTTTTA 3327

TAGTAGATGG TAAAATAAAA GAAGTTACAG ATATTGCAAT AAAGTATGGT CTAGA 3382

(2) INFORMATION ZU SEQ ID NO: 6:

(i) SEQUENZ CHARAKTERISTIKA:
(A) LÄNGE: 600 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 6:

Met Glu Phe Lys Ile Ile Ala Glu Tyr Phe Asp Arg Leu Glu Lys Ile
1               5                   10                  15

Ser Ser Arg Leu Gln Leu Thr Ser Leu Leu Ala Asp Leu Phe Lys Lys
            20                  25                  30

Thr Asp Lys Asn Val Ile Asp Lys Val Val Tyr Ile Ile Gln Gly Lys
        35                  40                  45

Leu Trp Pro Asp Phe Leu Gly Met Pro Glu Leu Gly Ile Gly Glu Lys
    50                  55                  60

Phe Leu Ile Arg Ala Leu Ser Ile Ala Thr Ser Val Ser Asp Asp Glu
65                  70                  75                  80

Ile Glu Lys Met Tyr Lys Ser Val Gly Asp Leu Gly Gln Val Ala Phe
                85                  90                  95

Asp Ile Lys Gln Lys Gln Gln Ser Ala Ser Ile Leu Ala Phe Leu Gly
            100                 105                 110

Ala Gln Lys Ala Ser Lys Pro Leu Thr Val Glu Lys Val Tyr Asp Asp
        115                 120                 125

Leu Ala Lys Val Ala Thr Ser Thr Gly Glu Gly Ser Arg Asp Ile Lys
    130                 135                 140

Ile Arg Leu Leu Ala Gly Leu Leu Lys Asp Ala Ser Pro Leu Glu Ala
145                 150                 155                 160

Lys Tyr Leu Val Arg Phe Val Asp Gly Arg Leu Arg Val Gly Ile Gly
                165                 170                 175

Asp Ala Thr Ile Leu Asp Ala Leu Ala Ile Thr Phe Gly Gly Gly Gln
            180                 185                 190

Asn Phe Arg Pro Ile Val Glu Arg Ala Tyr Asn Leu Arg Ala Asp Leu
        195                 200                 205

Gly Asn Ile Ala Lys Ile Leu Ala Asn Gly Gly Ile Glu Gln Leu Lys
    210                 215                 220

Asn Ile Lys Pro Gln Pro Gly Ile Pro Ile Arg Pro Met Leu Ala Glu
225                 230                 235                 240

Arg Leu Ser Asp Pro Ala Glu Met Leu Ser Lys Val Gly Asn Ile Ala
                245                 250                 255

Leu Val Asp Tyr Lys Tyr Asp Gly Glu Arg Gly Gln Ile His Lys Ala
            260                 265                 270

Gly Asp Lys Ile Phe Ile Phe Ser Arg Arg Leu Glu Asn Ile Thr Asn
        275                 280                 285

Gln Tyr Pro Asp Val Ala Glu Tyr Ile Ser Lys Tyr Val Lys Gly Asn
    290                 295                 300

Glu Phe Ile Val Glu Gly Glu Ile Ile Pro Val Asp Pro Glu Thr Gly
305                 310                 315                 320

Glu Met Arg Pro Phe Gln Glu Leu Met His Arg Lys Arg Lys Ser Asp
                325                 330                 335

Ile His Glu Ala Ile Lys Glu Tyr Pro Val Asn Val Phe Leu Phe Asp
            340                 345                 350

Leu Met Tyr Tyr Glu Gly Glu Asp Tyr Thr Val Lys Pro Leu Ser Glu
        355                 360                 365

Arg Arg Lys Lys Leu Glu Ser Ile Val Glu Asp Asn Asp Tyr Val His
    370                 375                 380

Ile Ala Thr [?]is Ile Ile Thr Asp Asn Val Glu Lys Leu Lys Glu Phe
385 390 395 400

Phe Tyr Gln Ala Ile Ser Glu Gly Ala Glu Gly Val Met Val Lys Ser
405 410 415

Leu Ala Pro Asp Ala Ile Tyr Gln Ala Gly Ser Arg Gly Trp Leu Trp
420 425 430

Ile Lys Phe Lys Arg Asp Tyr Gln Ser Glu Met Ala Asp Thr Val Asp
435 440 445

Leu Val Met Val Gly Ala Phe His Gly Lys Gly Arg Lys Gly Gly Lys
450 455 460

Tyr Ser Ser Phe Leu Met Ala Ala Tyr Asn Pro Asp Lys Asp Val Phe
465                 470                 475                 480

Glu Thr Val Cys Lys Val Ala Ser Gly Phe Thr Asp Ala Glu Leu Asp
                485                 490                 495

Asp Leu Gln Lys Lys Ile Ala Glu Leu Lys Arg Asp Thr Pro His Pro
            500                 505                 510

Arg Val Val Ser Thr Met Val Pro Asp Val Trp Leu Thr Pro Ala Leu
        515                 520                 525

Val Ala Glu Ile Ile Gly Ala Glu Ile Thr Ile Ser Pro Leu His Thr
    530                 535                 540

Cys Cys Lys Asp Gln Tyr Ala Glu Gly Gly Leu Ser Ile Arg Phe Pro
545                 550                 555                 560

Arg Phe Ile Arg Trp Arg Pro Asp Lys Ser Pro Glu Asp Ala Thr Thr
                565                 570                 575

Asn Arg Glu Ile Leu Glu Met Tyr Lys Ser Gln Leu Lys Lys Ile Glu
            580                 585                 590

Glu Lys Pro Ser Asp Gln Ser Val
        595                 600

(2) INFORMATION ZU SEQ ID NO: 7:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 7:

ACTTTCGCCA TTGGAAT 17

(2) INFORMATION ZU SEQ ID NO: 8:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 8:

CTCTTAAATC GACCCCG 17

(2) INFORMATION ZU SEQ ID NO: 9:

(i) SEQUENZ CHARAKTERISTIKA:
(A) LÄNGE: 38 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 9:

GCGGATCCAT CGAAGGTAGA ATGGAGTTTA AAATTATA 38

(2) INFORMATION ZU SEQ ID NO: 10:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 10:

TATGGATCCT TATACACTCT GATCTGACGG 30

(2) INFORMATION ZU SEQ ID NO: 11:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 11:

GTTTTCCCAG TCACGAC 17

(2) INFORMATION ZU SEQ ID NO: 12:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 12:

CACACAGGAA ACAGCTAT 18

(2) INFORMATION ZU SEQ ID NO: 13:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 13:

ATTAACCCTC ACTAAAG 17

(2) INFORMATION ZU SEQ ID NO: 14:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 14:

AATACGACTC ACTATAG 17